# EUROPÄISCHE PATENTSCHRIFT

(11) **EP 3 612 316 B1**
(45) Veröffentlichungstag und Bekanntmachung des Hinweises auf die Patenterteilung: **10.05.2023**
(21) Anmeldenummer: 18719152.3
(22) Anmeldetag: 19.04.2018
(51) Int. Cl.: A45D 34/02, B05B 5/043, B05B 5/053, A61M 35/00, B05B 9/08, B05B 1/14, B05B 5/16, B05B 9/04

(54) **ELEKTROSTATISCHER ZERSTÄUBER FÜR FLÜSSIGKEITEN**
ELECTROSTATIC ATOMISER FOR LIQUIDS
PULVÉRISATEUR ÉLECTROSTATIQUE POUR PULVÉRISER DES LIQUIDES

(30) Priorität: 21.04.2017 DE 102017108613
(43) Veröffentlichungstag der Anmeldung: 26.02.2020
(73) Patentinhaber: J. Wagner GmbH, 88677 Markdorf (DE)
(72) Erfinder: BARTHELMES, Jan, 88682 Salem (DE); GÖHRING, Alfred, 88682 Salem (DE); JELTSCH, Thomas, 88048 Friedrichshafen (DE); STOHL, Holger, 88677 Markdorf (DE); BISCHOFBERGER, Urban, 9442 Berneck (CH)
(74) Vertreter: Otten, Roth, Dobler & Partner mbB Patentanwälte
(86) Internationale Anmeldenummer: PCT/EP2018/060120
(87) Internationale Veröffentlichungsnummer: WO 2018/193068

(56) Entgegenhaltungen:
- EP-A1- 1 504 823
- EP-A1- 2 025 411
- WO-A2-2016/057942
- CN-U- 204 656 234
- US-A- 4 467 961
- US-A1- 2015 297 776
- US-A1- 2016 022 011

## Beschreibung

Die Erfindung betrifft einen elektrostatischen Zerstäuber für Flüssigkeiten nach dem Oberbegriff des Patentanspruchs 1.

Aus dem Stand der Technik, beispielsweise aus der US 2010/0116897 A1, kennt man bereits elektrostatische Zerstäuber für Flüssigkeiten. Der bekannte Zerstäuber weist den Nachteil auf, dass individuelle Bedürfnisse eines Benutzers, insbesondere basierend auf Benutzerdaten, beim Betrieb des Zerstäubers nicht berücksichtigt werden können.

Weiterhin ist aus der US 2016/0022011 A1 eine Vorrichtung zur Behandlung menschlicher Haut bekannt, welche ein äußeres Gehäuse mit einem greifbaren Abschnitt und einem Applikatorabschnitt, einen Applikatorkopf, mindestens eine Düse in dem Applikatorabschnitt mit einer Hauptachse zum Abgeben einer Hautbehandlungszusammensetzung durch eine Öffnung in dem Applikatorkopf auf die menschliche Haut, eine Bilderfassungsvorrichtung, die Bilder der menschlichen Haut durch die Öffnung aufnimmt, einen Prozessor, der die Bilder der menschlichen Haut analysiert, um Hautabweichungen zu identifizieren umfasst, wobei der Applikatorkopf vom äußeren Gehäuse abnehmbar ist, um das Ersetzen einer Patrone zu erleichtern, die die Hautbehandlungszusammensetzung enthält.

Aufgabe der Erfindung ist es, einen elektrostatischen Zerstäuber für Flüssigkeiten bereitzustellen, der unter Berücksichtigung individueller Bedürfnisse eines Benutzers sehr zuverlässig und flexibel bzw. anpassungsfähig betreibbar ist.

Diese Aufgabe wird durch einen elektrostatischen Zerstäuber gemäß Patentanspruch 1 gelöst. Weitere Ausgestaltungen der Erfindung sind Gegenstand von Unteransprüchen.

Elektrostatische Zerstäubung umfasst im Sinne der vorliegenden Erfindung alle Zerstäubungsvorgänge, welche Flüssigkeiten mit Effekten der Einwirkung einer Hochspannung zerstäuben. Insbesondere auch elektrohydrodynamische Effekte sowie elektrokinetische Effekte sind von dem Begriff dieser Art von Zerstäubung umfasst. Im Sinne der Erfindung kann unter einer elektrostatischen Zerstäubung auch eine elektrohydrodynamische Zerstäubung aufgefasst werden.

Es wird ein elektrostatischer Zerstäuber für Flüssigkeiten, insbesondere Kosmetika vorgeschlagen, welcher wenigstens ein Gehäuse, eine elektrische Energiequelle, ein Aktivierungsmittel, eine Kontrollelektronik, eine Hochspannungsquelle, einen Flüssigkeitstank, eine Fördervorrichtung sowie Zerstäuberdüsen umfasst, wobei die Kontrollelektronik und die Hochspannungsquelle in einem Innenraum des Gehäuses angeordnet sind.

Das Aktivierungsmittel im weiteren Sinne kann als Taster, aber auch als kapazitiver Schalter, als Reed Kontakt oder als Hall Sensor ausgebildet werden. Ergänzend oder alternativ kann ein Aktivierungsmittel in Form eines Hauptschalters, z.B. ein Gehäuseschieber vorgesehen werden oder eine Funktionsfreigabe kann über ein über eine Sende- und Empfangseinheit verbundenes Gerät, z.B. ein Smartphone erfolgen.

Erfindungsgemäß ist vorgesehen, dass die Kontrollelektronik Sensormittel umfasst und entsprechend den übrigen Merkmalen des Anspruchs 1 ausgeführt ist. Durch die Verwendung von Sensormitteln können benutzerspezifische Daten bei Betrieb des Zerstäubers berücksichtigt werden. Individuellen Bedürfnissen eines Benutzers beim Betrieb bzw. Verwenden des Zerstäubers kann man damit gerecht werden.

Durch eine entsprechende Sensorik kann die Beschichtungsmenge, und damit ggf. indirekt die Schichtdicke des mit dem Zerstäuber aufgebrachten Fluids ermittelt, oder zumindest abgeschätzt werden, so dass Rückschlüsse über den Beschichtungserfolg erzielt werden können. Dabei wäre ggf. auch eine Erfassung der Bewegung des Zerstäubers und/oder des Abstands zur beschichteten Oberfläche vorteilhaft hinzuzuziehen.

Der Begriff des Sensors ist dabei weit zu fassen. Grundsätzlich sind Sensoren auch als Erfassungsvorrichtungen von physikalischen Parametern wie z.B. Stromfluss, Spannung, Temperatur, Lichtintensität usw. anzusehen. Sensoren können aber auch Bewegungssensoren in Form von Gyro-Sensoren sein oder es können Lage und Beschleunigung erfasst werden. Auch Optische Sensoren wie z.B. Helligkeitssensoren oder dergleichen sind umfasst.

Das Sende-Empfangsmodul dient zur Kommunikation mit externen Geräten. Ob zur Kommunikation Standards wie Wireless-Lan, Bluetooth oder Mobiles Internet (z.B. LTE) eingesetzt wird, ist dem jeweiligen Anwendungsfall überlassen.

Zudem ist ein erfindungsgemäßer Zerstäuber zuverlässig und individuell betreibbar, da Sensormittel bzw. Sende- und/oder Empfangsmodul Daten zum Betrieb bzw. auch während des Betriebs bereitstellen können und insbesondere bei Änderungen von Daten den Betrieb des Zerstäubers entsprechend berücksichtigen bzw. anpassen können. Damit weist der Zerstäuber hinsichtlich seiner Verwendung auch eine hohe Flexibilität bzw. Anpassungsfähigkeit auf.

Der Erfindungsgemäße Zerstäuber kann für eine Vielzahl von Betriebsarten vorgesehen werden. Dabei kann z.B. eine Anwendung von Sonnenschutzmittel in ähnlicher Weise zum Einsatz kommen, wie die Aufbringung von Insektenschutz oder eines Deodorants. Auch industrielle Anwendungen mit Farben oder Lacken wären denkbar.

Eine bevorzugte Ausgestaltung der Erfindung kann vorsehen, dass wenigstens eines der Sensormittel zur Erfassung von Spannung und/oder Stromstärke der Hochspannungsquelle, insbesondere der Belastung der Hochspannungsquelleausgebildet ist. Auf diese Weise kann z.B. die Leistung der Fördervorrichtung beeinflusst und/oder ausgewertet werden, wodurch das Sprühergebnis beinflussbar ist.

Um die Leistung der Fördervorrichtung zu beeinflussen und insbesondere die Abgabemenge von Flüssigkeit aus den Zerstäuberdüsen regulieren zu können, kann in einer vorteilhaften Ausgestaltung der Erfindung vorgesehen sein, dass wenigstens eines der Sensormittel zur Erfassung von Intensität und/oder Wellenlänge von einfallendem Licht ausgebildet ist.

Im Anwendungsbeispiel des Aufbringens von Sonnenschutzfluiden wäre durch einen UV Sensor eine Berechnung möglich, wann der Sonnenschutz aufgefrischt werden sollte. Dabei können nicht nur Umgebungsdaten wie Intensität des Sonnenlichtes und Temperatur einfließen, sondern es können auch individuelle Profile für den jeweiligen Nutzer definiert werden, welche einen betreffenden Hauttyp oder eine Vorbelastung der Haut, z.B. durch einen Sonnenbrand, in Betracht ziehen

Zur weiteren Beeinflussung bzw. Regelbarkeit der Leistung der Fördervorrichtung insbesondere auch im Hinblick auf eine abzugebende Flüssigkeitsmenge aus den Zerstäuberdüsen bzw. der Temperatur austretender Flüssigkeit kann die Erfindung vorsehen, dass wenigstens eines der Sensormittel zur Erfassung von Lufttemperatur und/oder Luftfeuchtigkeit der Umgebung des Zerstäubers ausgebildet ist.

Um den Betrieb des Zerstäubers unter Berücksichtigung von benutzerspezifischen Daten ermöglichen zu können, kann in einer vorteilhaften Weiterbildung der Erfindung vorsehen sein, dass wenigstens ein Sende- und/oder Empfangsmodul über eine Funkverbindung mit einem Mobiltelefon oder Tablet oder einem ähnlichen Computer verbindbar ist.

Auf diese Weise kann eine Personalisierung der Betriebsart des Zerstäubers erfolgen. Beispielsweise kann im Anwendungsfall des Sonnenschutzes der konkrete Hauttyp im Smartphone ermittelt, gemessen oder durch externe Daten gespeichert werden. Sodann kann die Auftragung des Sonnenschutzes durch den Zerstäuber genau auf den Hauttyp abgestimmt erfolgen, wobei auch zusätzliche Umwelteinflüsse, wie z.B. Temperatur und Luftfeuchtigkeit berücksichtigt werden können, falls diese das Auftragungsergebnis beeinflussen.

Um den Zerstäuber z.B. auch als frei transportables Gerät nutzen zu können und damit eine flexiblere Nutzung des Zerstäubers an verschiedenen Orten zu ermöglichen, kann die Erfindung in einer Ausgestaltung auch vorsehen, dass die Energiequelle als aufladbarer elektrischer Speicher (Akku) ausgebildet ist und der Zerstäuber wenigstens eine Ladevorrichtung zum Laden der Energiequelle umfasst, wobei die Ladevorrichtung insbesondere als normierter Anschluss, vorzugsweise als USB-Anschluss ausgebildet ist. Aufladbare Energiequellen weisen zudem eine hohe Lebensdauer auf. Normierte Anschlüsse sind kostengünstig erhältlich und entsprechende Ladetechnik ist hoch verfügbar.

Eine weitere vorteilhafte Weiterbildung der Erfindung kann vorsehen, dass die Energiequelle als aufladbarer elektrischer Speicher (Akku) ausgebildet ist und der Zerstäuber wenigstens eine Ladevorrichtung zum Laden der Energiequelle umfasst, wobei die Ladevorrichtung als elektromagnetische Spule einer induktiven Ladeeinrichtung ausgebildet ist, wobei die Spule vorzugsweise innerhalb des Gehäuses entweder an einem Kopfabschnitt oder an einem Griffabschnitt angeordnet ist. Eine derartige Lademöglichkeit mittels Induktion weist den Vorteil auf, dass der Zerstäuber ohne Einstecken oder Entfernen einer elektrischen Leitung, z.B. eines Kabels, nach vor oder nach dem Laden z.B. von einer Ladestation entfernt werden kann. Zudem kann durch Fernbleiben von entsprechend angeordneten Öffnungen zum Anschluss von Ladeleitungen, etc. ein Eindringen von Feuchtigkeit bzw. Schmutz, etc. in solche Öffnungen unterbunden werden. Dadurch kann die Lebensdauer des Zerstäubers erhöht werden.

Um ein Sprühergebnis zu erzielen, welches individuellen Bedürfnissen eines Benutzers entspricht, kann eine überdies vorteilhafte Weiterbildung der Erfindung vorsehen, dass wenigstens ein Sensormittel zur Erfassung einer aus dem Flüssigkeitstank entnommenen Flüssigkeitsmenge oder zur Erfassung eines Füllstandes von im Flüssigkeitstank befindlicher Flüssigkeit ausgebildet ist.

Der erfindungsgemäße Zerstäuber ist hinsichtlich einer Berücksichtigung individueller Bedürfnisse noch vielseitiger einsetzbar, wenn die Kontrollelektronik mehrere Sensormittel zur Erfassung von Lufttemperatur und/oder Luftfeuchtigkeit der Umgebung des Zerstäubers und zur Erfassung von Intensität und/oder Wellenlänge von einfallendem Licht und zur Erfassung von Spannung und/oder Stromstärke umfasst.

Durch intelligente Berücksichtigung der Daten mehrerer Sensoren, z.B. Abgegebener Menge, Bewegungsmuster, Abstand von der Oberfläche sowie der Belastung durch Sonnenstrahlen können z.B. im Anwendungsfall des Sonnenschutzes optimierte und persönlich abgestimmte Anwendungsszenarien in Menge und Häufigkeit erzielt werden.

Eine Ausführungsform des erfindungsgemäßen Zerstäubers sieht vor, dass über die Kontrollelektronik ein Speichermittel, insbesondere eine RFID Markierung an dem Flüssigkeitstank auslesbar und/oder beschreibbar ist. Durch das Speichermittel kann zum einen der Flüssigkeitstank identifiziert werden und es besteht die Möglichkeit, falls das Speichermittel auch flexibel beschreibbar ist, nutzungsspezifische Daten wie z.B. den Tag der ersten Öffnung oder eine Restfüllmenge zu speichern, zu aktualisieren oder für weitere Geräte auslesbar zu machen. So können Flüssigkeitstanks nachhaltig austauschbar gestaltet werden. Auch ein Wechsel zwischen unterschiedlichen Zerstäubergeräten ist dadurch ermöglicht.

Die Kontrollelektronik umfasst mehrere Sensormittel zur Erfassung einer Lage und/oder eine Bewegung im Raum umfasst, wobei über die Kontrollelektronik Lageänderungen und/oder Bewegungsabläufe erfassbar, speicherbar und analysierbar sind, können Benutzungsdaten des individuellen Benutzers erfasst und ausgewertet werden. Insbesondere in Zusammenwirkung mit einem externen Gerät, z.B. einem Smartphone kann ein Bewegungsmuster einen Rückschluss über die besprühten Stellen bzw. Flächen zulassen.

Erfindungsgemäß ist auch ein Verfahren zum Betrieb eines oben beschriebenen Zerstäubers vorgesehen. Bei dem Verfahren ist vorgesehen, dass die Kontrollelektronik Sensordaten von mindestens einem Sensor über wenigstens ein Sende- und/oder Empfangsmodul an ein externes Gerät übergibt und das externe Gerät die Daten verarbeitet und/oder darstellt und/oder weiterleitet, wobei es vorgesehen ist, dass die Kontrollelektronik über wenigstens ein Sende- und/oder Empfangsmodul von einem externen Gerät Daten erhält und die Kontrollelektronik diese Daten verarbeitet und/oder darstellt und/oder weiterleitet, insbesondere zur Bereitstellung von Betriebszuständen oder Geräteparametern nutzt.

Auch die Nutzung von Daten des externen Geräts, z.B. des Smartphones, beispielsweise über erfasste Beschichtungserfolge oder Analysen der Bedürfnisse des Nutzers, können an den Zerstäuber rückgemeldet werden, so dass dieser seine Betriebszustände oder Parameter automatisch anpasst.

Durch dieses Verfahren ist es z.B. möglich, Füllstände des Flüssigkeitstanks auf einem Smartphone anzuzeigen, die eingestellten Geräteparameter zu übertragen und zu personalisieren oder erfasste Bewegungsdaten zu verarbeiten. Auch die Nutzung, insbesondere die Häufigkeit und Intensität der Nutzung des Zerstäubers kann übergeben und z.B. an eine Cloud oder eine Datenbank weitergeleitet werden.

Bei einer weiteren Ausführungsform des Verfahrens ist vorgesehen, dass die Kontrollelektronik über wenigstens ein Sende- und/oder Empfangsmodul von einem externen Gerät Sensordaten von mindestens einem Sensor erhält und die Kontrollelektronik diese Daten verarbeitet und/oder darstellt und/oder weiterleitet. Sensordaten eines externen Geräts, z.B. ein Temperatursensor oder dergleichen, können ebenfalls an den Zerstäuber übergeben werden, so dass dieser diese Daten nutzen und den Betriebszustand dahingehend anpassen kann. Wird der Zerstäuber z.B. an einem Ort betrieben, welcher im Falle der Sonnenschutzanwendung eine erhebliche Nutzungsfrequenz fordert (starke Sonnenbelastung) so kann die Position durch den GPS Sensor des Smartphones ermittelt werden, und aus den üblichen Daten für diese Region eine Steuerung der abgegebenen Flüssigkeitsmenge am Zerstäuber erfolgen.

Vergleichbar kann das Verfahren zum Betrieb eines Zerstäubers auch dahingehend genutzt werden, dass die Kontrollelektronik Daten über Betriebszustände und/oder Geräteparameter über wenigstens ein Sende- und/oder Empfangsmodul an ein externes Gerät übergibt und das externe Gerät die Daten verarbeitet und/oder darstellt und/oder weiterleitet.

Ein Smartphone kann die Nutzungshäufigkeit und die Entnahmemenge aus dem Flüssigkeitstank protokollieren und auswerten. Erfolgt eine zu geringe Nutzung, so kann z.B. durch eine Applikation auf dem externen Gerät, z.B. dem Smartphone eine Signalisierung erfolgen, dass eine erneute Nutzung empfohlen wird.

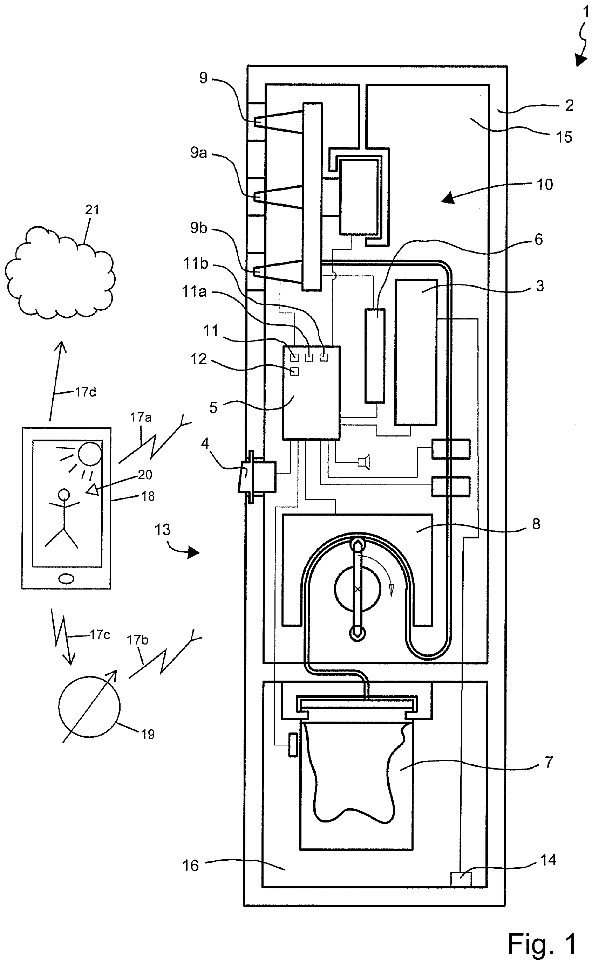

Im Folgenden wird die Erfindung anhand einer Zeichnung näher erläutert, woraus weitere erfindungswesentliche Merkmale und Vorteile der Erfindung hervorgehen. Die einzige Figur 1 zeigt in stark vereinfachter schematischer Darstellung einen erfindungsgemäßen Zerstäuber.

Der elektrostatische Zerstäuber 1 umfasst ein Gehäuse 2 mit einem Innenraum 10, in welchem Gehäuse 2 eine elektrische Energiequelle 3, ein Aktivierungsmittel 4, eine Kontrollelektronik 5, eine Hochspannungsquelle 6, ein Flüssigkeitstank 7, eine Fördervorrichtung 8 und Zerstäuberdüsen 9, 9a, 9b angeordnet sind.

Im gezeigten Ausführungsbeispiel umfasst die Kontrollelektronik 5 Sensormittel 11, 11a, 11b und ein Sende- und/oder Empfangsmodul 12.

Sensormittel 11, 11a, 11b können vorgesehen bzw. ausgestaltet sein, um Spannung und/oder Stromstärke bzw. Intensität und/oder Wellenlänge von einfallendem Licht bzw. Lufttemperatur und/oder Luftfeuchtigkeit der Umgebung 13 des Zerstäubers 1 erfassen zu können. Auf diese Weise entsprechend ermittelte Daten können z.B. eine Förderleistung der Fördervorrichtung 8 und damit auch ein Sprühergebnis des Zerstäubers 1 beeinflussen.

Sensormittel 11, 11a, 11b können auch vorgesehen bzw. ausgestaltet sein, um Entfernungen zwischen Zerstäuberdüsen 9, 9a, 9b und einem zu besprühenden Objekt (nicht gezeigt) durch Feldanalyse zu ermitteln bzw. zu detektieren.

Sensormittel 11, 11a, 11b können auch vorgesehen bzw. ausgestaltet sein, um eine Fläche eines besprühten Objekts ermitteln bzw. detektieren zu können.

Weiterhin können Sensormittel 11, 11a, 11b vorgesehen bzw. ausgestaltet sein, um optische Analysen eines zu besprühenden Objekts durchführen zu können.

Sensormittel 11, 11a, 11b können auch vorgesehen bzw. entsprechend ausgestaltet sein, um wiederholte Anwendungen, z.B. Mehrfachanwendungen detektieren und/oder diese unterbinden zu können.

Sensormittel 11, 11a, 11b können auch vorgesehen bzw. ausgestaltet sein, um eine Ausrichtung der Zerstäuberdüsen 9, 9a, 9b im Hinblick auf ein zu besprühendes Objekt ermitteln zu können, beispielsweise durch Vergleich der jeweiligen Potentiale der Zerstäuberdüsen 9, 9a, 9b.

Das Sende- und/oder Empfangsmodul 12 kann z.B. vorgesehen sein, um über eine Funkverbindung mit einem Mobiltelefon oder Tablet (nicht dargestellt) verbunden zu werden. Hierbei kann beispielsweise auch vorgesehen sein, dass Daten eines Benutzers, z.B. Körpergewicht, Körpergröße von einem Mobiltelefon oder Tablet zu dem Zerstäuber 1 übertragen werden und entsprechend angepasste Sprühvorgänge durchgeführt werden. Solche Sprühvorgänge können dann individuell durchgeführt werden und z.B. auch nur einzelne Körperpartien eines Benutzers betreffen.

Mittels einer entsprechenden Überwachung der Sprühaktivität des Zerstäubers im Hinblick auf gewählte Anforderungen durch Sensorelemente 11, 11a, 11b bzw. Sende- und/oder Empfangsmodul 12 kann ein festgelegtes Sprühergebnis ggf. korrigiert werden. Hierbei können insbesondere Zeit und versprühte Menge der Flüssigkeit überwacht werden und ggf. weitere Sprühvorgänge durchgeführt werden. Eine Überwachung bzw. eine Überprüfung von Sprühvorgängen kann auch auf der Basis von Bilddaten erfolgen, die beispielsweise von einem Smartphone oder Tablet an den Zerstäuber 1 übermittelt werden.

Der Zerstäuber 1 umfasst im gezeigten Ausführungsbeispiel eine elektrische Energiequelle 3, die aufladbar ist. Hierzu umfasst der Zerstäuber 1 eine Ladevorrichtung 14, die in einem Griffbereich 16 des Zerstäubers innerhalb bzw. in einem Innenraum 10 des Gehäuses 2 angeordnet ist.

Über das Sende-/Empfangsmodul 12 kann über eine Funkverbindung 17a, z.B. WLan oder Bluetooth, eine Verbindung zu einem Externen Gerät, z.B. einem Smartphone 18 oder über eine Funkverbindung 17b zu einem externen Sensor 19 hergestellt werden. Auch eine gleichzeitige Verbindung zu mehreren Geräten, insbesondere Sensoren 19 ist denkbar.

Die Daten der internen Sensormittel 11a und 11b und/oder der externen Sensoren 19 und/oder des Smartphones 18 können dann im Smartphone 18 in einer Applikation 20 verarbeitet werden und z.B. auf dem Bildschirm Anweisungen und Empfehlungen für den Benutzer ausgeben. Auch eine Fernsteuerung des Zerstäubers 1 ausgehend von dem Smartphone 18 ist denkbar.

Es besteht weiterhin die Möglichkeit, dass auch das Smartphone 18 direkt über eine Funkverbindung 17c mit einem Sensor verbunden ist, und dessen Daten auswertet und z.B. UV-Werte an die Applikation 20 und damit an den Zerstäuber 1 weitergibt.

Überdies ist vorgesehen, dass Daten über eine Funkverbindung 17d an ein externes Rechenzentrum, z.B. eine Cloud 21 übergeben werden, um Nutzerprofile zu speichern oder statistische Auswertungen vorzunehmen.

Im Sinne der Erfindung kann unter einer elektrostatischen Zerstäubung auch eine elektrohydrodynamische Zerstäubung aufgefasst werden.

Im Sinne der Erfindung ist unter einer Flüssigkeit jedwedes Liquid aufzufassen. Im Sinne der Erfindung kann die Flüssigkeit ein Kosmetikum, auch flüssige Farbe oder Lack oder dergleichen sein.

### Bezugszeichenliste:

- 1: Zerstäuber
- 2: Gehäuse
- 3: elektrische Energiequelle
- 4: Aktivierungsmittel
- 5: Kontrollelektronik
- 6: Hochspannungsquelle
- 7: Flüssigkeitstank
- 8: Fördervorrichtung
- 9: Zerstäuberdüse
- 9a: Zerstäuberdüse
- 9b: Zerstäuberdüse
- 10: Innenraum
- 11: Sensormittel
- 11a: Sensormittel
- 11b: Sensormittel
- 12: Sende- und/oder Empfangsmodul
- 13: Umgebung (des Zerstäubers)
- 14: Ladevorrichtung
- 15: Kopfabschnitt
- 16: Griffabschnitt
- 17a: Funkverbindung Smartphone - Zerstäuber
- 17b: Funkverbindung Sensor - Zerstäuber
- 17c: Funkverbindung Smartphone - Sensor
- 17d: Funkverbindung Smartphone - Cloud
- 18: Smartphone
- 19: Sensor
- 20: Applikation
- 21: Cloud / Internet

## Patentansprüche

1. Elektrostatischer Zerstäuber (1) für Flüssigkeiten, insbesondere Kosmetika, wenigstens umfassend
- ein Gehäuse (2),
- eine elektrische Energiequelle (3),
- ein Aktivierungsmittel (4),
eine Kontrollelektronik (5),
- eine Hochspannungsquelle (6),
- einen Flüssigkeitstank (7),
- eine Fördervorrichtung (8)
- Zerstäuberdüsen (9, 9a, 9b),
- wobei die Kontrollelektronik (5) und die Hochspannungsquelle (6) in einem Innenraum (10) des Gehäuses (2) angeordnet sind,
**dadurch gekennzeichnet, dass**
die Kontrollelektronik (5) Sensormittel (11) umfasst, wobei sie mehrere Sensormittel zur Erfassung einer Lage und/oder einer Bewegung im Raum umfasst, wobei über die Kontrollelektronik Lageänderungen und/oder Bewegungsabläufe erfassbar, speicherbar und analysierbar sind.

2. Zerstäuber nach Anspruch 1, **dadurch gekennzeichnet, dass** wenigstens eines der Sensormittel (11) zur Erfassung von Spannung und/oder Stromstärke der Hochspannungsquelle (6) insbesondere zur Erfassung der Belastung der Hochspannungsquelle ausgebildet ist.

3. Zerstäuber nach einem der vorangegangenen Ansprüche, **dadurch gekennzeichnet, dass** wenigstens eines der Sensormittel (11) zur Erfassung von Intensität und/oder Wellenlänge von einfallendem Licht ausgebildet ist.

4. Zerstäuber nach einem der vorangegangenen Ansprüche, **dadurch gekennzeichnet, dass** wenigstens eines der Sensormittel (11) zur Erfassung von Lufttemperatur und/oder Luftfeuchtigkeit der Umgebung (13) des Zerstäubers (1) ausgebildet ist.

5. Zerstäuber nach einem der vorangegangenen Ansprüche, **dadurch gekennzeichnet, dass** wenigstens ein Sende- und/oder Empfangsmodul (12) über eine Funkverbindung mit einem Mobiltelefon oder Tablet verbindbar ist.

6. Zerstäuber nach einem der vorangegangenen Ansprüche, **dadurch gekennzeichnet, dass** die Energiequelle (3) als aufladbarer elektrischer Speicher ausgebildet ist und der Zerstäuber (1) wenigstens eine Ladevorrichtung (14) zum Laden der Energiequelle (3) umfasst, wobei die Ladevorrichtung (14) insbesondere als normierter Anschluss, vorzugsweise als USB-Anschluss ausgebildet ist.

7. Zerstäuber nach einem der Ansprüche 1-5, **dadurch gekennzeichnet, dass** die Energiequelle (3) als aufladbarer elektrischer Speicher ausgebildet ist und der Zerstäuber (1) wenigstens eine Ladevorrichtung (14) zum Laden der Energiequelle (3) umfasst, wobei die Ladevorrichtung (14) als elektromagnetische Spule einer induktiven Ladeeinrichtung ausgebildet ist, wobei die Spule vorzugsweise innerhalb des Gehäuses (2) entweder an einem Kopfabschnitt (15) oder an einem Griffabschnitt (16) angeordnet ist

8. Zerstäuber nach einem der vorangegangenen Ansprüche, **dadurch gekennzeichnet, dass** wenigstens ein Sensormittel (11) zur Erfassung einer aus dem Flüssigkeitstank (7) entnommenen Flüssigkeitsmenge oder zur Erfassung eines Füllstandes von im Flüssigkeitstank (7) befindlicher Flüssigkeit ausgebildet ist.

9. Zerstäuber nach einem der vorangegangenen Ansprüche, **dadurch gekennzeichnet, dass** über die Kontrollelektronik (5) ein Speichermittel, insbesondere eine RFID Markierung an dem Flüssigkeitstank (7) auslesbar und/oder beschreibbar ist.

10. Zerstäuber nach einem der vorangegangenen Ansprüche, **dadurch gekennzeichnet, dass** die Kontrollelektronik (5) mehrere Sensormittel (11, 11a, 11b)
- zur Erfassung von Lufttemperatur und/oder Luftfeuchtigkeit der Umgebung (13) des Zerstäubers (1) und
- zur Erfassung von Intensität und/oder Wellenlänge von einfallendem Licht und
- zur Erfassung von Spannung und/oder Stromstärke umfasst.

11. Verfahren zum Betrieb eines Zerstäubers nach einem der vorangegangenen Ansprüche, **dadurch gekennzeichnet, dass** die Kontrollelektronik (5) Sensordaten von mindestens einem Sensor über wenigstens ein Sende- und/oder Empfangsmodul (12) an ein externes Gerät übergibt und das externe Gerät die Daten verarbeitet und/oder darstellt und/oder weiterleitet.

12. Verfahren nach dem vorangegangenen Anspruch,
**dadurch gekennzeichnet, dass** die Kontrollelektronik (5) über wenigstens ein Sende- und/oder Empfangsmodul (12) von einem externen Gerät Sensordaten von mindestens einem Sensor erhält und die Kontrollelektronik diese Daten verarbeitet und/oder darstellt und/oder weiterleitet.

13. Verfahren nach einem der vorangegangenen Ansprüche 11 und 12,
**dadurch gekennzeichnet, dass** die Kontrollelektronik (5) Daten über Betriebszustände und/oder Geräteparameter über wenigstens ein Sende- und/oder Empfangsmodul (12) an ein externes Gerät übergibt und das externe Gerät die Daten verarbeitet und/oder darstellt und/oder weiterleitet.

14. Verfahren nach einem der vorangegangenen Ansprüche 11-13,
**dadurch gekennzeichnet, dass** die Kontrollelektronik (5) über wenigstens ein Sende- und/oder Empfangsmodul (12) von einem externen Gerät Daten erhält und die Kontrollelektronik diese Daten verarbeitet und/oder darstellt und/oder weiterleitet, insbesondere zur Bereitstellung von Betriebszuständen oder Geräteparametern nutzt.

## Claims

1. Electrostatic atomizer (1) for liquids, in particular cosmetics, at least comprising
- a housing (2),
- an electrical energy source (3),
- an activation means (4),
- control electronics (5),
- a high-voltage source (6),
- a liquid tank (7),
- a delivery device (8),
- atomizer nozzles (9, 9a, 9b),
- the control electronics (5) and the high-voltage source (6) being arranged in an interior space (10) of the housing (2),
**characterized in that**
the control electronics (5) comprise sensor means (11), these comprising a number of sensor means for detecting a position and/or a movement in space, with the control electronics making it possible for changes in position and/or movement sequences to be detected, stored and analyzed.

2. Atomizer according to Claim 1, **characterized in that** at least one of the sensor means (11) is formed for detecting the voltage and/or the current intensity of the high-voltage source (6), in particular for detecting the loading of the high-voltage source.

3. Atomizer according to either of the preceding claims, **characterized in that** at least one of the sensor means (11) is formed for detecting the intensity and/or the wavelength of incident light.

4. Atomizer according to one of the preceding claims, **characterized in that** at least one of the sensor means (11) is formed for detecting the air temperature and/or the humidity of the air in the surrounding area (13) of the atomizer (1).

5. Atomizer according to one of the preceding claims, **characterized in that** at least one transmitting and/or receiving module (12) can be connected via a radio link to a mobile phone or tablet.

6. Atomizer according to one of the preceding claims, **characterized in that** the energy source (3) is formed as a chargeable electrical store and the atomizer (1) comprises at least one charging device (14) for charging the energy source (3), the charging device (14) being formed in particular as a standardized connection, preferably as a USB connection.

7. Atomizer according to one of Claims 1-5, **characterized in that** the energy source (3) is formed as a chargeable electrical store and the atomizer (1) comprises at least one charging device (14) for charging the energy source (3), while the charging device (14) is formed as an electromagnetic coil of an inductive charging device, the coil preferably being arranged within the housing (2), either on a head portion (15) or on a grip portion (16).

8. Atomizer according to one of the preceding claims, **characterized in that** at least one sensor means (11) is formed for detecting an amount of liquid taken from the liquid tank (7) or for detecting a filling level of liquid in the liquid tank (7).

9. Atomizer according to one of the preceding claims, **characterized in that** a memory means, in particular an RFID marking on the liquid tank (7), can be read and/or written by the control electronics (5).

10. Atomizer according to one of the preceding claims, **characterized in that** the control electronics (5) comprise a number of sensor means (11, 11a, 11b)
- for detecting the temperature and/or humidity of the air in the surrounding area (13) of the atomizer (1) and
- for detecting the intensity and/or the wavelength of incident light and
- for detecting the voltage and/or the current intensity.

11. Method for operating an atomizer according to one of the preceding claims, **characterized in that** the control electronics (5) transfer sensor data from at least one sensor via at least one transmitting and/or receiving module (12) to an external device and the external device processes the data and/or displays them and/or passes them on.

12. Method according to the preceding claim, **characterized in that** the control electronics (5) obtain sensor data of at least one sensor via at least one transmitting and/or receiving module (12) from an external device, and the control electronics process and/or display and/or pass on these data.

13. Method according to either of the preceding Claims 11 and 12, **characterized in that** the control electronics (5) transfer data concerning operating states and/or device parameters via at least one transmitting and/or receiving module (12) to an external device and the external device processes and/or displays and/or passes on the data.

14. Method according to one of the preceding Claims 11-13, **characterized in that** the control electronics (5) obtain data via at least one transmitting and/or receiving module (12) from an external device and the control electronics process and/or display and/or pass on these data, in particular use them for providing operating states or device parameters.

## Revendications

1. Pulvérisateur électrostatique (1) pour liquides, en particulier pour cosmétiques, comprenant au moins
- un boîtier (2),
- une source d'énergie électrique (3),
- un moyen d'activation (4),
- une électronique de commande (5),
- une source de haute tension (6),
- un réservoir de liquide (7),
- un dispositif de transport (8),
- des buses de pulvérisation (9, 9a, 9b),
- dans lequel l'électronique de commande (5) et la source de haute tension (6) sont disposées dans un espace intérieur (10) du boîtier (2),
**caractérisé en ce que**
l'électronique de commande (5) comprend des moyens de détection (11), dans lequel elle comprend plusieurs moyens de détection pour détecter une position et/ou un mouvement dans l'espace, dans lequel des modifications de position et/ou des séquences de mouvement peuvent être détectées, mémorisées et analysées par l'intermédiaire de l'électronique de commande.

2. Pulvérisateur selon la revendication 1, **caractérisé en ce qu'**au moins l'un des moyens de détection (11) est réalisé de manière à détecter la tension et/ou l'intensité du courant de la source de haute tension (6), en particulier pour détecter la charge de la source de haute tension.

3. Pulvérisateur selon l'une quelconque des revendications précédentes, **caractérisé en ce qu'**au moins l'un des moyens de détection (11) est réalisé de manière à détecter l'intensité et/ou la longueur d'onde de la lumière incidente.

4. Pulvérisateur selon l'une quelconque des revendications précédentes, **caractérisé en ce qu'**au moins l'un des moyens de détection (11) est réalisé de manière à détecter la température de l'air et/ou l'humidité de l'air dans l'environnement (13) du pulvérisateur (1).

5. Pulvérisateur selon l'une quelconque des revendications précédentes, **caractérisé en ce qu'**au moins un module d'émission et/ou de réception (12) peut être relié à un téléphone mobile ou à une tablette par l'intermédiaire d'une liaison radio.

6. Pulvérisateur selon l'une quelconque des revendications précédentes, **caractérisé en ce que** la source d'énergie (3) est réalisée sous la forme d'un accumulateur électrique rechargeable et **en ce que** le pulvérisateur (1) comprend au moins un dispositif de charge (14) pour charger la source d'énergie (3), dans lequel le dispositif de charge (14) est réalisé en particulier sous la forme d'une connexion normalisée, de préférence une connexion USB.

7. Pulvérisateur selon l'une quelconque des revendications 1-5, **caractérisé en ce que** la source d'énergie (3) est réalisée sous la forme d'un accumulateur électrique rechargeable et **en ce que** le pulvérisateur (1) comprend au moins un dispositif de charge (14) pour charger la source d'énergie (3), dans lequel le dispositif de charge (14) est réalisé sous la forme d'une bobine électromagnétique d'un dispositif de charge à induction, dans lequel la bobine est de préférence disposée à l'intérieur du boîtier (2), soit sur une partie formant tête (15), soit sur une partie formant poignée (16).

8. Pulvérisateur selon l'une quelconque des revendications précédentes, **caractérisé en ce qu'**au moins un moyen de détection (11) est réalisé de manière à détecter une quantité de liquide prélevée dans le réservoir de liquide (7) ou à détecter un niveau du liquide se trouvant dans le réservoir de liquide (7).

9. Pulvérisateur selon l'une quelconque des revendications précédentes, **caractérisé en ce qu'**un moyen de mémorisation, en particulier un marquage RFID peut être lu et/ou écrit sur le réservoir de liquide (7) au moyen de l'électronique de commande (5).

10. Pulvérisateur selon l'une quelconque des revendications précédentes, **caractérisé en ce que** l'électronique de commande (5) comporte plusieurs moyens de détection (11, 11a, 11b)
- pour détecter la température de l'air et/ou l'humidité de l'air dans l'environnement (13) du pulvérisateur (1) et
- pour détecter l'intensité et/ou la longueur d'onde de la lumière incidente et
- pour détecter la tension et/ou l'intensité du courant.

11. Procédé de fonctionnement d'un pulvérisateur selon l'une quelconque des revendications précédentes, **caractérisé en ce que** l'électronique de commande (5) transmet des données de capteur d'au moins un capteur à un appareil externe par l'intermédiaire d'au moins un module d'émission et/ou de réception (12) et **en ce que** l'appareil externe traite et/ou visualise et/ou transmet les données.

12. Procédé selon la revendication précédente, **caractérisé en ce que** l'électronique de commande (5) reçoit en provenance d'un appareil externe, par l'intermédiaire d'au moins un module d'émission et/ou de réception (12), des données de capteur d'au moins un capteur et **en ce que** l'électronique de commande traite et/ou visualise et/ou transmet ces données.

13. Procédé selon l'une quelconque des revendications 11 et 12 précédentes, **caractérisé en ce que** l'électronique de commande (5) transmet à un appareil externe, par l'intermédiaire d'au moins un module d'émission et/ou de réception (12), des données relatives à des états de fonctionnement et/ou à des paramètres de l'appareil et **en ce que** l'appareil externe traite et/ou visualise et/ou transmet les données.

14. Procédé de fonctionnement d'un pulvérisateur selon l'une quelconque des revendications 11-13 précédentes, **caractérisé en ce que** l'électronique de commande (5) reçoit des données en provenance d'un appareil externe, par l'intermédiaire d'au moins un module d'émission et/ou de réception (12) et **en ce que** l'électronique de commande traite et/ou visualise et/ou transmet ces données, et les utilise en particulier pour fournir des états de fonctionnement ou des paramètres de l'appareil.
